(19) **Europäisches Patentamt / European Patent Office / Office européen des brevets**

(11) **EP 2 210 583 A1**

(12) # EUROPEAN PATENT APPLICATION
published in accordance with Art. 153(4) EPC

(43) Date of publication:
**28.07.2010 Bulletin 2010/30**

(21) Application number: **08844299.1**

(22) Date of filing: **16.10.2008**

(51) Int Cl.:
*A61K 8/73* (2006.01)  *A61K 8/02* (2006.01)
*A61K 8/19* (2006.01)  *A61K 8/20* (2006.01)
*A61K 8/34* (2006.01)  *A61K 8/65* (2006.01)
*A61K 8/67* (2006.01)  *A61K 8/86* (2006.01)
*A61K 9/70* (2006.01)  *A61K 33/06* (2006.01)
*A61K 47/10* (2006.01)  *A61K 47/34* (2006.01)
*A61K 47/36* (2006.01)  *A61K 47/42* (2006.01)
*A61L 15/16* (2006.01)  *A61L 15/58* (2006.01)
*A61P 17/16* (2006.01)  *A61Q 19/00* (2006.01)

(86) International application number:
**PCT/JP2008/068752**

(87) International publication number:
**WO 2009/057456 (07.05.2009 Gazette 2009/19)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **31.10.2007 JP 2007284489**

(71) Applicant: **FUJIFILM Corporation
Tokyo 106-8620 (JP)**

(72) Inventor: **TSUJIHATA, Shigetomo
Ashigarakami-gun
Kanagawa 258-8577 (JP)**

(74) Representative: **HOFFMANN EITLE
Patent- und Rechtsanwälte
Arabellastrasse 4
81925 München (DE)**

(54) **GEL SHEET AND COSMETIC PREPARATION IN SHEET FORM USING THE SAME**

(57)     A gel sheet including a hydrogel, the hydrogel including at least 50 parts by mass of water-soluble divalent metal salt with respect to 100 parts by mass of anionic polymer compound is provided.

EP 2 210 583 A1

**Description**

TECHNICAL FIELD

The present invention relates to a gel sheet for use in the fields of pharmaceuticals, quasi-drugs, cosmetics, hygiene products, sundries, and the like, and a cosmetic preparation in sheet form including the gel sheet.

BACKGROUND ART

Gel sheets are products obtained by forming gels having a high water content into sheets. Owing to their high water retention capability, gel sheets can be used for various applications for continuously supplying water and the like to areas where the gel sheets are placed.

Gel sheets may suitably be used, for example, for the following: facial masks, adhesive skin patches or the like used for beauty art, facial treatment, skin treatment or the like; carriers of active ingredients such as skin penetrating components or antiphlogistic analgesic components; adhesive tapes for a living body and wound-dressings which are used for protection of wounds, fixation of drugs, and the like; and the like. These gel sheets are adhered to the skin to protect the skin surface and impart water retention to the skin surface. In addition, when a gel sheet contains various active ingredients and is tightly adhered to the skin, the gel sheet exerts functions such as controlling skin temperature, imparting water retention, or supplying the active ingredients in the gel sheet to a living body. In particular, when a gel sheet containing a liquid component is tightly adhered to the skin and left in this state for a certain period of time, the physiological activity of the skin can be enhanced owing to the increase in water content or temperature, for example,, and the enhancement in physiological activity leads to further improvement in the penetration into the skin of the active ingredients contained in the gel sheet.

As gel sheets having such actions, gel sheets are conventionally known which contain, as constituent components, polysaccharides such as collagen, chitin, chitosan, alginic acid or cellulose as a gel base material, in consideration of use on a living body (see, for example, Japanese Patent Application Laid-Open (JP-A) No. 3-81213).

Furthermore, from the viewpoint of improving the functionality of a gel sheet, a technique for enhancing a bleeding effect from a gel base material using a particular alkylene oxide derivative is known (see, for example, JP-A 2005-225837), and a technique of enhancing a solution exuding effect by adding a polyethylene glycol or an electrolyte is known (see, for example, JP-A 2003-183147), in order to improve moisture retention. Both techniques are aimed to improve the penetration of the water or active ingredients contained in the gel sheet into the skin.

In order to rapidly supply the water and active ingredients contained in the gel sheet to a target area, what is important is not only the water retention capability of the gel sheet, but the syneresis property for supplying the water or the like retained by the gel sheet to a target area in the adjacent body to which the gel sheet is adhered is also important. However, in the above techniques, the syneresis property is insufficient in terms of, for example, achieving rapid and efficient penetration of the active ingredients and the like contained in the hydrogel into the skin while maintaining capability of moisture retention at the skin surface.

DISCLOSURE OF THE INVENTION

Problem to be solved by the invention

Objects of the present invention, which has been made in view of the above, are to provide a gel sheet which has excellent handleability, and which efficiently provides water and/or active ingredient contained in a hydrogel to an adjacent body to which the gel sheet is adhered (adhesion target) when applied to, mainly, living bodies and contacted with the adjacent adhesion target such as skin, and also to provide a cosmetic preparation in sheet form which includes the gel sheet.

Means for solving the problems

As a result of intensive study by the inventor, the inventor has found that the above objects are solved by using a hydrogel which includes at least a water-soluble divalent metal salt and an anionic polymer compound, and in which the amount of the water-soluble divalent metal salt is at least half of the amount of the anionic polymer compound, and has completed the present invention.

Configurations of the present invention are described hereinafter.

<1> A gel sheet comprising a hydrogel, the hydrogel comprising at least 50 parts by mass of water-soluble divalent metal salt with respect to 100 parts by mass of anionic polymer compound.

<2> The gel sheet according to <1>, wherein the content of the water-soluble divalent metal salt in the hydrogel is from 0.2% by mass to 10% by mass.

<3> The gel sheet according to <1> or <2>, wherein a content of a tri- or higher-valent metal salt contained in the hydrogel is 0.1 % by mass or lower.

<4> The gel sheet according to any one of <1> to <3>, wherein the hydrogel comprises at least one selected from the group consisting of collagen and collagen-degradation products.

<5> The gel sheet according to any one of <1> to <4>, wherein the hydrogel comprises a polyether.

<6> The gel sheet according to any one of <1> to <5>, wherein the hydrogel comprises an O/W emulsion.

<7> The gel sheet according to any one of <1> to <6>, wherein the hydrogel comprises a polysaccharide.

<8> The gel sheet according to any one of <1> to <7>, wherein the hydrogel comprises a polyhydric alcohol compound.

<9> The gel sheet according to any one of <1> to <8>, wherein the hydrogel has a water content ratio of from 70 to 95% by mass.

<10> The gel sheet according to any one of <1> to <9>, wherein the gel sheet comprises: a hydrogel layer comprising the hydrogel; and a sheet-shaped base material that is arranged within the hydrogel layer or adjacent to the hydrogel layer.

<11> The gel sheet according to <10>, wherein the hydrogel layer has a thickness of from 0.4 to 2 mm.

<12> A cosmetic preparation in sheet form, comprising the gel sheet according to any one of <1> to <11>.

The gel sheet of the invention includes a hydrogel comprising at least 50 parts of water-soluble divalent metal salt with respect to 100 parts of anionic polymer compound that is a gel base material, and therefore the hydrogel included in the gel sheet exhibits excellent syneresis properties. In other words, due to the contact with an adjacent adhesion target to which the gel sheet is adhered, for example skin if the get sheet is attached to a living body, water and/or active ingredient are efficiently provided to the horny layer.

Furthermore, the hydrogel included in the gel sheet of the invention retains a large amount of aqueous components. The gel sheet of the invention exhibits excellent handleability since the sheet is flexible, is easily deformed by stress, and does not easily break when handled due to a high gel strength thereof. Further, the gel sheet of the invention exhibits excellent adhesion to an adhesion target since the surface of the gel sheet is in a moistened state. The "adhesion target" as used herein has a function of absorbing an aqueous phase component included in the hydrogel and the active ingredient(s) and the like dissolved or dispersed therein.

The "syneresis property" as used in the invention refers to a function whereby the aqueous phase component included in the hydrogel and various active ingredients dissolved or dispersed therein exude from the hydrogel and transfer to a living body or absorber which serves as an adjacent adhesion target. Thus, even if the hydrogel itself is capable of efficiently retaining the aqueous component, a low syneresis property will result in insufficiency of the function of supplying water and/or active ingredients to the adjacent adhesion target such as a living body.

It is a significant feature that, since the gel sheet of the invention has excellent syneresis property, not only can the aqueous phase component, such as water, retained in the hydrogel be supplied to the adhesion target from the hydrogel, but the active ingredient(s) dissolved or dispersed in the aqueous phase component, such as various water-soluble active ingredients, emulsion particles of an oil phase component, nanocolloid, nanoparticles, or the like, can also be provided to the adhesion target from the hydrogel.

The syneresis property in the invention may be evaluated based on, for example, a syneresis rate calculated by the following method.

(Determination of syneresis rate)

A test piece having a predetermined size is prepared by being cut out of a gel sheet. The test piece is put between two upper sheets and two lower sheets of filter paper each having an area larger than that of the test piece, and left to stand under an atmosphere of 25°C and 55% relative humidity for ten minutes. The change in mass of the filter paper is measured to determine the amount of liquid absorbed by the filter paper, and a syneresis rate is calculated in accordance with the following Formula 1 (unit: % by mass).

$$\text{Syneresis rate} = (\text{amount of liquid absorbed by filter paper / initial mass of gel}) \times 100$$

(Formula 1)

As described above, the gel sheet of the invention can efficiently provide water and/or active ingredient(s) to an adjacent adhesion target that is capable of absorbing the water and/or active ingredient(s), and may be preferably used for any applications in which continuous supply and/or penetration of water is required. Furthermore, since the gel sheet of the invention is highly biocompatible and highly safe, the effects thereof is remarkable particularly when used for applications in which the gel sheet is applied to a living body to efficiently provide water and/or various active ingredients.

The cosmetic preparation in sheet form of the invention comprises the gel sheet.

The "gel sheet" as used in the invention refers to a shaped product in sheet form, which contains a hydrogel. The gel sheet may be a sheet consisting only of a hydrogel layer in which a hydrogel is formed into a sheet, or may be a sheet including a hydrogel layer and a sheet-shaped base material which is provided for the purpose of reinforcement, protection of active ingredients, improvement in handleability, or the like. The scope of "gel sheet" encompasses facial masks or adhesive skin patches used for beauty art, facial treatment, skin treatment or the like; carriers of active ingredients such as skin penetrating components or antiphlogistic analgesic components; adhesive tapes for living bodies and wound-dressings which are used for protection of wounds, fixation of drugs, and the like; and the like. The "gel sheet" is suitable for an adhesive sheet for a living body which is used by being directly adhered to the skin for the purpose of retention, penetration into the skin, or the like of the active ingredient(s) and/or water. The adhesive gel sheet for a living body is useful as a cosmetic preparation in sheet form such as a facial mask which is to be adhered to the skin to provide water and/or active ingredient(s) to the skin.

Effect of the Invention

According to the invention, there is provided a gel sheet which has excellent handleability, and which efficiently provide water and/or active ingredient contained in a hydrogel to an adjacent adhesion target when applied to, mainly, living bodies and contacted with the adjacent adhesion target such as skin, and a cosmetic preparation in sheet form which includes the gel sheet is also provided.

BEST MODE FOR CARRYING OUT THE INVENTION

Hereinafter, the present invention is described in detail.

The gel sheet of the invention includes an anionic polymer compound and a water-soluble divalent metal salt, and, optionally, various additives and active ingredient(s) which may be additionally used, wherein the addition amount of water-soluble divalent metal salt in the preparation of a hydrogel is at least 50 parts by mass with respect to 100 parts by mass of anionic polymer compound.

Hereinafter, respective components which can be used in the gel sheet of the invention are described sequentially.

<Water-soluble divalent metal salt>

Examples of the divalent metal which forms the water-soluble divalent metal salt to be used in the invention include magnesium, calcium, strontium, and barium, which belong to Group II metals of the periodic table, and $Cu^{2+}$, $Fe^{2+}$, $Zn^{2+}$, and $Mn^{2+}$, which are transition metals. Water-soluble salts of magnesium or calcium are preferable. The term "water-soluble" as used herein means a solubility of at least 0.1% by mass in pure water at 25°C.
The water-soluble salt may be either an inorganic salt or an organic salt, and examples thereof include, though not being particularly limited, inorganic salts such as hydrochlorates, nitrates, sulfurates, phosphates, and carbonates; salts of organic acids such as citrates, lactates, malates, succinates, ascorbates, and gluconates; and organic-inorganic combined salts such as ascorbic acid phosphate ester salts.
Of these, magnesium chloride, calcium chloride, magnesium lactate, magnesium malate, magnesium citrate, calcium citrate, magnesium ascorbate, calcium ascorbate, magnesium ascorbyl phosphate, magnesium gluconate, and calcium gluconate are more preferable, and magnesium chloride and magnesium ascorbyl phosphate are particularly preferable.

The amount of water-soluble divalent metal salt to be added when the gel sheet of the invention is formed has to be at least 50 parts with respect to 100 parts by mass of anionic polymer compound described below (hereinafter, "part(s) by mass" may be referred to "part(s)" in the specification), and is preferably from 50 parts to 1,000 parts, more preferably from 70 parts to 500 parts, and particularly preferably from 80 to 300 parts, from the viewpoint of the effects.

Furthermore, the content of water-soluble divalent metal salt in the prepared hydrogel is preferably from 0.1 to 5% by mass, more preferably from 0.2 to 4% by mass, and particularly preferably from 0.5 to 2% by mass. When the proportion of water-soluble metal salt is within the above range, syneresis property from the hydrogel is improved, and a gel sheet having excellent handleability can be obtained. In this regard, in an embodiment, the content of water-soluble divalent metal salt in the prepared hydrogel may be, for example, from 0.2 to 10% by mass.

In the hydrogel formed from the anionic polymer and the water-soluble divalent metal salt, the water-soluble divalent metal salt is used for forming the gel, and does not necessarily exist in a form of "salt". The content of the water-soluble divalent salt or of a compound derived therefrom in the prepared hydrogel may be determined by measuring the amount of the metal that constitutes the metal salt, and calculating the content from the obtained amount in consideration of the counter ion. This procedure allows determination of the amount of water-soluble divalent metal salt used in the formation of the prepared hydrogel.

<Anionic polymer compound>

The anionic polymer compound to be used in the invention is not particularly limited as long as the compound is a polymer compound having, in a molecule thereof, an anionic group selected from, for example, a carboxyl group, a sulfo group, a phospho group, or the like, and may be a synthetic polymer or a natural polymer.

In the hydrogel, the anionic polymer compound may be used singly, or two or more thereof may be used in mixture.

Examples of synthetic anionic polymer compounds include acrylic acid (co)polymers, methacrylic acid (co)polymers, maleic acid (co)polymers, itaconic acid (co)polymers, p-vinylbenzoate (co)polymers, 2-acrylamide-2-methyl-1-propanesulfonic acid (co)polymers, and styrenesulfonic acid (co)polymers.

Examples of natural anionic polymer compounds include pectinic acid, alginic acid, agar, carrageenan, fucoidan, hyaluronic acid, condroitin sulfate, heparin, gellan gum, native gellan gum, xanthan gum, carboxymethylcellulose, carboxymethyl starch, carboxymethyldextran, and polyglutamic acid. Moreover, DNAs and RNAs may be used as anionic polymers in the invention.

Of these, from the viewpoint of simultaneously achieving water retention capability, gel strength, and syneresis property, acrylic acid (co)polymers, alginic acid, agar, carrageenan, hyaluronic acid, gellan gum, native gellan gum, xanthan gum, and carboxymethylcellulose are preferable, and agar, carrageenan, gellan gum, native gellan gum, and xanthan gum are particularly preferable.

From the viewpoint of enhancing gel strength, the anionic polymer compound has a molecular weight preferably in a range of from 10,000 to 5,000,000, and more preferably in a range or from 20,000 to 2,000,000.

In the gel sheet of the invention, the content of anionic polymer compound with respect to the hydrogel is preferably from 0.01 to 10% by mass, more preferably from 0.1 to 8% by mass, and particularly preferably from 0.2 to 4% by mass. When the content is within the above range, a gel strength providing a good handleability may be achieved, and a hydrogel which has a sufficient syneresis property can be obtained.

When the anionic polymer compound and the water-soluble divalent metal salt are used in combination, it is thought that a three-dimensional network structure having a weakly-crosslinked structure is formed in the resultant hydrogel system, and a sufficient liquid component-retaining property and a good syneresis property are exhibited.

The combination of the anionic polymer compound and the water-soluble divalent metal salt to be contained in the hydrogel may be appropriately selected in accordance with the purpose. From the viewpoint of simultaneously achieving the gel strength and the syneresis property, preferable examples of the combination of the water-soluble divalent metal salt/anionic polymer compound include, but are not limited to, those shown below. Magnesium ascorbyl phosphate/carrageenan,

Magnesium ascorbyl phosphate/gellan gum,

Magnesium ascorbyl phosphate/agar,

Magnesium ascorbyl phosphate/xanthan gum,

Magnesium ascorbyl phosphate/acrylic acid (co)polymer,

Magnesium ascorbyl phosphate/carboxymethylcellulose,

Magnesium chloride/carrageenan,

Magnesium chloride/gellan gum,

Magnesium chloride/acrylic acid (co)polymer,

Magnesium lactate/carrageenan,

Magnesium lactate/agar

Calcium lactate/gellan gum

Calcium ascorbate/carrageenan

Calcium gluconate/gellan gum.

<Tri- or higher-valent metal salt>

In the invention, the content of tri- or higher-valent metal salt in the hydrogel is preferably 0.1% by mass or lower from the viewpoint of preventing a decrease in syneresis property.

Examples of tri- or higher-valent metal salts include a salt containing a polyvalent cation such as $Al^{3+}$, $Fe^{3+}$, $Ti^{3+}$, $Ti^{4+}$, $In^{3+}$, $Zr^{4+}$, or $Ta^{5+}$. Specific examples thereof include potassium alum, ammonium alum, iron alum, aluminum sulfate, polyaluminum chloride, synthetic aluminum silicate, aluminum hydroxide, aluminum stearate, aluminum acetate, ferric sulfate, ferric hydroxide, titanium lactate, and zirconium acetate.

The content ratio of tri- or higher-valent metal salt in the hydrogel of the invention is preferably 0.1% by mass or lower, more preferably 0.05% by mass or lower, and particularly preferably 0.01% by mass or lower, and it is most preferable that the hydrogel is free from the polyvalent metal salt except unavoidable impurities. When the content of trivalent metal salt exceeds 0.1% by mass, syneresis property may decrease.

Although the hydrogel of the invention has to comprise the anionic polymer compound and the water-soluble divalent metal salt, the hydrogel may further comprise other various compounds, as desired, in accordance with the purpose of use of the gel sheet.

Hereinafter, additional components which may be included in the hydrogel of the invention are described.

<Polyether>

In the gel sheet of the invention, the hydrogel preferably contains a polyether for the purpose of improving syneresis property.

Examples of the polyether include polyethylene oxide, polypropylene oxide, polyethylene oxide/polypropylene oxide block copolymers, and polyethylene oxide/polypropylene oxide/polyethylene oxide block copolymers, and block polymers containing polyethylene oxide and polypropylene oxide are particularly preferable. Specifically, polyethylene oxide/polypropylene oxide block copolymers (hereinafter may also be referred to "PEO-PPO block polymers") or polyethylene oxide/polypropylene oxide/polyethylene oxide block copolymers (hereinafter may also be referred to "PEO-PPO-PEO block polymers") are particularly preferable. These polyethers are also known as poloxamers, and are easily obtainable as commercially-available products under the names of "PLURONIC" and "LUTROL" (both manufactured by BASF), NEWPOL (manufactured by Sanyo Chemical Industries, Ltd.), EPAN (manufactured by Dai-ichi Kogyo Seiyaku Co., Ltd.), PRONON (manufactured by NOF Corporation) and the like.

The polyether which can be used in the invention has a weight average molecular weight (Mw) of preferably from 1,000 to 1,000,000, and more preferably from 5,000 to 500,000.

When the polyether is used, the content of the polyether in the hydrogel is preferably from 0.05 to 20% by mass, more preferably from 0.1 to 10% by mass, and particularly preferably from 0.2 to 5% by mass.

Within the above range, when the content of the polyether is 0.05% by mass or higher, an effect of improving skin penetration property of the active ingredient(s) added to the hydrogel is sufficiently achieved, and when the content is 20% by mass or lower, a significant decrease in storage stability is prevented.

<Polysaccharides>

In the gel sheet of the invention, it is preferable to further add, into the hydrogel, polysaccharides other than the anionic polysaccharides described as examples of the anionic polymer compound, for improvement in handleability and from the viewpoint of improving gel strength.

Examples of polysaccharides that can be used in the invention include neutral polysaccharides (for example, cellulose, amylose, amylopectin, dextran, pullulan, inulin, galactan, mannan, xylan, arabinan, glucomannan, galactomannan, agarose, methylcellulose, hydroxypropylcellulose, curdlan, and xyloglucan) and cationic polysaccharides (such as chitin, chitosan, cationized cellulose, cationized starch, and cationized dextran).

Of these, polysaccharides efficient in viscosity increase and gelation are more preferable, and glucomannan, galactomannan, agarose, methylcellulose, and hydroxypropylcellulose are particularly preferable. Furthermore, in order to improve gelation property thereof, two or more of the polysaccharides may be used in combination.

The content of polysaccharide with respect to the hydrogel in the invention is preferably from 0.01 to 5% by mass, more preferably from 0.1 to 4% by mass, and particularly preferably from 0.2 to 2% by mass.

When the content of polysaccharide is within the above range, a gel-strength improving effect and favorable handleability are attained, as well as decrease in syneresis property and decrease in skin penetration property of the aqueous components are prevented.

<Polyhydric alcohol compound>

In the gel sheet of the invention, it is preferable that the hydrogel further contains a polyhydric alcohol compound from the viewpoint of the skin penetration property of the active ingredient(s) or storage stability. Specific examples of the polyhydric alcohol compound include glycerins (such as glycerin and diglycerin), glycols (for example, diethylene glycol, triethylene glycol, tetraethylene glycol, polyethylene glycol, 1,2-propanediol, 1,3-propanediol, dipropylene glycol, 1,2-butanediol, 1,3-butanediol, 1,2-pentanediol, 1,2-hexanediol, and 1,2-octanediol), saccharides (such as glucose, fructose, mannose, galactose, xylose, arabinose, glucosamine, N-acetylglucosamine, sucrose, lactose, maltose, isomaltose, trehalose, cellobiose, kojibiose, sophorose, maltotriose, raffinose, and stachyose), and sugar alcohols (such as glycerol, threitol, erythritol, arabinitol, xylitol, ribitol, mannitol, sorbitol, galactitol, and inositol). The polyhydric alcohol compound may be used singly, or two or more thereof may be used in mixture.
Of these, glycerins or glycols are preferable for use in the gel sheet of the invention, and, in particular, glycerin, 1,2-propanediol, 1,2-butanediol, 1,2-pentanediol, 1,2-hexanediol, and 1,3-butanediol are more preferable. Furthermore, it is particularly preferable to use glycerin, 1,2-propanediol, 1,3-butanediol, or 1,2-hexanediol.
The proportion of polyhydric alcohol compound in the hydrogel of the invention is preferably 50% by mass or lower, and particularly preferably from 1 to 20% by mass.
When the proportion of polyhydric alcohol compound in the hydrogel of the invention is 50% by mass or lower, decrease in gel strength is prevented, and handleability is improved.

<Hydrophilic polymer>

In the adhesive gel sheet for a living body used in the invention, a known hydrophilic polymer may be added to the hydrogel in order to improve moisture retention capability, as long as dissolution property when adhered to the skin is not impaired. A hydrophilic polymer may be used also for improving dimensional stability of the adhesive gel sheet for a living body.
Examples of hydrophilic polymers which may be used in the invention include synthetic polymers and natural polymers, each of which is a polymer other than the anionic polymer compound and polysaccharide described above and has a hydrophilic functional group (for example, a hydroxyl group, a carbamoyl group, an amino group, an ammonio group, or an ethyleneoxy group). The hydrophilic polymer may be used singly, or two or more hydrophilic polymers may be used in mixture.
Examples of synthetic polymers having a hydrophilic group suitable for the invention include vinyl alcohol (co) polymers, 2-hydroxyethyl acrylate (co)polymers, acrylamide (co)polymers, acryloylmorpholine (co)polymers, N-vinylpyrrolidone (co)polymers, vinylamine (co)polymers, N,N-dimethyl diallyl ammonium chloride (co)polymers, 2-methacryloyloxyethyl ammonium chloride (co)polymers, polyethylene glycol methacrylate (co)polymers, and polyethyleneimine.
From the viewpoint of moisture retention capability, the hydrophilic polymer has a weight average molecular weight of preferably from 1,000 to 500,000, and more preferably from 5,000 to 100,000.
From the viewpoints of moisture retention capability and handleability, the content of the hydrophilic polymer is preferably from 0.05 to 5% by mass, and more preferably from 0.1 to 3% by mass, relative to the hydrogel.

<Excipient>

In the gel sheet of the invention, an excipient may further be added to the hydrogel in order to improve dimensional stability. Organic or inorganic fine particles may preferably be used as the excipient. The organic fine particles are preferably known polystyrene particles, polymethacrylate particles, or microcrystalline cellulose. The inorganic fine particles are preferably titanium oxide, silica, alumina, calcium carbonate, kaolin, a clay mineral or the like. Of these, silica or a clay mineral is preferable, and vapor-phase process silica or synthetic smectite having an average particle diameter of 200 nm or less is particularly preferable.
The proportion of excipient in the hydrogel of the invention is preferably 10% by mass or lower, more preferably 5% by mass or lower, and particularly preferably 2% by mass or lower.

<Water content ratio>

The water content ratio of the hydrogel in the gel sheet of the invention is preferably from 70% by mass to 95% by mass. When the water content ratio is within the range, not only the releasing efficiency of the active ingredient(s) from the hydrogel is improved, but irritation to the skin when the gel sheet is adhered thereto is decreased.
The water content ratio of the hydrogel is preferably from 70% by mass to 95% by mass, more preferably from 75% by mass to 95% by mass, and particularly preferably from 80% by mass to 90% by mass. When the water content ratio is less than 70% by mass, the skin penetration property of the components may decrease. When the water content ratio exceeds 95% by mass, the strength of the hydrogel decreases, and, therefore, handleability may decrease.

The water content ratio of the hydrogel can be measured based on the ratio of mass decrease that occurs when drying by heating or drying under reduced pressure. Specifically, 1 g of hydrogel is taken out of a gel sheet and dried under reduced pressure at 25°C until change in mass of the sample becomes unobservable, and a value calculated from the following Formula 2 is regarded as the water content ratio.

$$\text{Water content ratio (\% by mass)} = [(\text{initial mass} - \text{mass after drying})/\text{initial mass}] \times 100 \cdots (\text{Formula 2})$$

The water content ratio of the hydrogel in the present specification is measured according to the method described above. The water content ratio of the hydrogel can be measured also by using a moisture meter of Karl Fischer system, infrared ray system, or electrical resistance system. When the hydrogel formulation liquid itself gelates, the addition ratio of the water content may be regarded as the water content ratio of the gel.

Since the gel sheet of the invention has excellent syneresis property, inclusion of various active ingredients with a water content ratio within the above preferable range enables effective permeation of such active ingredients into a certain region of a desired adhesion target, for example, a living body. Accordingly, the gel sheet of the invention can be suitably used as a cosmetic preparation or a sustained-release carrier for the active ingredients.

The active ingredients are appropriately selected depending on the purpose of use of the gel sheet. Hereinafter, active ingredients which may be contained in the hydrogel of the invention are described, but active ingredients are not limited thereto.

<Collagen or degradation products thereof>

In the gel sheet of the invention, it is preferable that the hydrogel further contains at least one selected from collagen and degradation products thereof for the purpose of improving moisture retention capability. The collagen as used herein is not particularly limited, and various collagen extracts may be used. Extraction can be performed using a collagen-containing raw material and using a known technique such as acid solubilization, alkali solubilization, neutral salt solubilization, or enzyme solubilization. As the collagen-containing raw material, any material can be used as long as the raw material contains collagen, and examples thereof include skin or scale, bone, cartilage, tendon, and organs of vertebrates (for example, bovine, swine, sardine, and shark). Because of high collagen contents, bone, cartilage, skin or scale, tendon, placenta and the like are preferably used. Of these, water-soluble collagens are preferable as collagens suitable for use in the invention.

Collagen degradation products of the invention are obtained by hydrolysis of collagen using a proteolytic enzyme such as collagenase, trypsin, or chymotrypsin, an acid, or an alkali, or by denaturation by heating of collagen.

Examples of collagen degradation products include acid-processed gelatin, alkali-processed gelatin, enzymatically-degraded gelatin, collagen tripeptide, collagen dipeptide, and amino acids (such as glycine, proline, hydroxyproline, and acetyl hydroxyproline).

The content of collagen or collagen degradation product in the hydrogel of the invention is preferably from 0.05 to 20% by mass, more preferably from 0.1 to 10% by mass, and particularly preferably from 0.2 to 5% by mass. When the amount is 0.05% by mass or higher, a water retention effect is attained when adhered to the skin. When the amount is 20% by mass or lower, handleability is favorable.

<Oil/water emulsion>

In the gel sheet of the invention, the hydrogel preferably contains an oil-in-water emulsion (hereinafter may be referred to as O/W emulsion). Specifically, an O/W emulsion containing an oil-soluble medicinal ingredient is preferable. Since the gel sheet of the invention has excellent syneresis property, when the gel sheet is formed to have such a configuration, a fine emulsion containing the oil-soluble medicinal ingredient effectively permeates into the skin, leading to, for example, an enhanced effect in beautifying the skin.

Preferable examples of the oil-soluble medicinal ingredient include lipid-soluble vitamins and analogs thereof (such as tocopherol, tocotrienol, retinol, retinal, and calciferol), sterols (such as cholesterol and phytosterol), ubiquinone (such as CoQ-10), as well as sphingolipid, ceramide, orizanol, squalene, squalane, carotenoid, and derivatives thereof, and carotenoid is particularly preferable in the invention.

Examples of carotenoid include actinioerythrol, astaxanthin, bixin, canthaxanthin, capsanthin, β-8'-apo-carotenal, β-12'-apo-carotenal, α-carotene, β-carotene, γ-carotene, β-cryptoxanthin, lutein, lycopene, violerythrin, zeaxanthin, fucoxanthin, and derivatives thereof.

Of these, astaxanthin, lutein, zeaxanthin, and β-cryptoxanthin are preferable, and astaxanthin, which is known to have an antioxidant effect, an anti-inflammatory effect, a skin aging preventing effect, and a skin-lightening effect, is particularly preferable.

The O/W emulsion may contain one or more other components, such as an emulsifier, that can be generally included in respective phases of an emulsion composition, in generally-employed amounts. The scope of other components encompasses other components disclosed herein, such as polyhydric alcohols.

The volume average particle diameter of emulsified particles of the O/W emulsion is preferably from 1 to 200 nm, and particularly preferably from 1 to 150 nm.

The volume average particle diameter may be measured using a commercially-available particle size distribution analyzer or the like. Known methods of measuring particle diameter of the emulsion include optical microscopy, confocal laser microscopy, electron microscopy, atomic force microscopy, static light scattering method, laser diffractometry, dynamic light scattering method, centrifugal sedimentation method, electrical pulse measurement, chromatography, and ultrasonic attenuation method, and apparatuses corresponding to the respective mechanisms are commercially available.

From the viewpoints of the volume average particle diameter range in the invention and ease of measurement, a dynamic light scattering method is preferable for the measurement of the volume average particle diameter of the emulsion in the invention. Examples of commercially-available measurement apparatuses employing dynamic light scattering include a NANOTRAC UPA (Nikkiso Co., Ltd.), a dynamic light scattering particle size distribution analyzer LB-550 (Horiba Ltd.), and a fiber-optics particle analyzer FPAR-1000 (Otsuka Electronics Co., Ltd.).

In the invention, the volume average particle diameter of the O/W emulsion is a value measured using a fiber-optics particle analyzer FPAR-1000 (Otsuka Electronics Co., Ltd.) at 25°C.

In the measurement method of the volume average particle diameter, the oil phase is diluted with pure water so that the concentration of the oil phase falls within a range of from 0.1 to 1% by mass, and measurement is performed using a glass tube for measurement. The volume average particle diameter can be determined as a cumulative (50%) value obtained by a measurement in which a refractive index of the dispersion medium of 1.3313 (pure water) and a viscosity of the dispersion medium of 0.8846 mPs (pure water) are inputted.

The method of manufacturing the O/W emulsion is not particularly limited, and, for example, a method disclosed in Japanese Patent Application Laid-Open (JP-A) No. 2005-75817 may be used. Alternatively, it is preferable to manufacture the O/W emulsion by a method having the steps of a) dissolving a water-soluble emulsifier in an aqueous medium to obtain a water phase, b) mixing and dissolving carotenoid, tocopherol, lecithin, and, if necessary, another fat or oil to obtain an oil phase, and c) mixing the water phase and the oil phase under stirring, thereby performing emulsification and dispersion to obtain an emulsion composition.

In the emulsification and dispersion, it is particularly preferable to use two or more emulsification apparatuses in such a manner that, for example, a high pressure homogenizer is used after emulsification using a generally-used emulsification apparatus that utilizes shearing action, such as a stirrer, an impeller stirrer, a homomixer, or a continuous flow-shearing apparatus, is performed. When a high pressure homogenizer is used, liquid droplets of the fine particles of the emulsion can be further uniformized.

The content of the O/W emulsion relative to the hydrogel of the invention is preferably from 0.001 to 10% by mass, and more preferably from 0.05 to 1% by mass, from the viewpoints of efficacy and skin permeability of the active ingredients.

The content of oil-soluble medicinal ingredient in the hydrogel of the invention is, though varying depending on the medicinal ingredient, preferably from 0.0001 to 10% by mass, and more preferably from 0.005 to 5% by mass.

When the oil-soluble medicinal ingredient is a carotenoid, the amount thereof relative to the hydrogel of the invention is preferably from 0.0001 to 0.5% by mass, more preferably from 0.0005 to 0.1 % by mass, and particularly preferably from 0.001 to 0.05% by mass. When the content of a carotenoid is 0.0001% by mass or higher, effects (such as an effect in beautifying the skin) are sensed after the adhesive gel sheet for a living body of the invention is adhered to the skin. When the amount is 0.5% by mass or lower, coloring on the skin can be prevented and a discomfort feeling is less likely to be produced.

<Organic acid>

The hydrogel of the invention may further contain an organic acid from the viewpoint of controlling pH. Specific examples of salts of organic acids include acetic acid, α-hydroxy acids (for example, citric acid, lactic acid, gluconic acid, malic acid, and succinic acid), ascorbic acid, and pyrrolidone carboxylic acid. One of these may be used singly, or two or more thereof may be used in mixture. The content of organic acid in the hydrogel is preferably from 0.01 to 5% by mass, and more preferably from 0.05 to 2% by mass.

<Antiseptic agent>

It is preferable that the adhesive gel sheet of the invention further contains an antiseptic agent for the purpose of taking a countermeasure against deterioration caused by microorganisms. Examples of the antiseptic agent include phenol, benzoic acid and salts thereof, salicylic acid and salts thereof, p-oxybenzoic acid esters (such as methylparaben, ethylparaben, propylparaben, and butylparaben), 2-phenoxyethanol, dehydroacetic acid and salts thereof, sorbic acid and salts thereof, alkylaminoethyl glycine chloride, triclosan, benzalkonium chloride, ethanol, propanol, and butanol. One of these may be used singly, and combinational use thereof is more preferable. Of these, p-oxybenzoic acid esters and phenoxyethanol are particularly preferable.
The content of antiseptic agent in the hydrogel of the invention is preferably from 0.01 to 0.5% by mass, more preferably from 0.02 to 0.3% by mass, and particularly preferably from 0.03 to 0.2% by mass.

<Fragrance material>

To the hydrogel of the invention, a fragrance material may be added in order to improve a relaxing effect. Examples of the fragrance material include alcohol-based fragrance materials, phenol-based fragrance materials, carboxylic acid-based fragrance materials, and amine-based fragrance materials.
Examples of alcohol-based fragrance materials include leaf alcohol, 3-octenol, 9-decenol, linalool, geraniol, nerol, citronellol, rhodinol, dimethyloctanol, hydroxycitronellol, tetrahydrolinalool, lavanduol, mugol, myrcenol, terpineol, 1-menthol (L-menthol), borneol, isopulegol, tetrahydromugol, bornyl methoxy cyclohexanol, novol, farnesol, nerolidol, santalol, sandalol, cedrol, vetiverol, patchouli alcohol, benzyl alcohol, β-phenyl ethyl alcohol, γ-phenyl propyl alcohol, cinnamic alcohol, anisyl alcohol, α-amyl cinnamic alcohol, dimethyl benzyl carbinol, methyl phenyl carbinol, diemthyl phenyl carbinol, β-phenyl ethyl dimethyl carbinol, β-phenylethyl methyl ethyl carbinol, phenoxyethyl alcohol, phenyl glycol, and tertiary butyl cyclohexanol.
Examples of phenol-based fragrance materials include eugenol, vanillin, and hinokitiol. Examples of carboxylic acid-based fragrance materials include cinnamic acid, phenylacetic acid, and hydrocinnamic acid. Examples of amine-based fragrance materials include indole, skatole, 2-methyl tetrahydroquinoline, and 6-methylquinoline.

<Additives and the like>

Furthermore, various active ingredients, additives, and the like may be added depending on the purpose of use of the gel sheet of the invention. Examples of such active ingredients and additives include those described in the following.
Antioxidants (such as tocopherols, dibutyl hydroxy toluene, butyl hydroxy anisole, and gallic acid esters),
Ultraviolet absorbers (such as p-methoxycinnamic acid, octyl p-methoxycinnamate, 2-methoxy-2-hydroxybenzophenone, 2-ethylhexyl p-dimethylaminobenzoate),
pH adjusters (such as buffer agents such as lactic acid-sodium lactate, citric acid-sodium citrate, or succinic acid-sodium succinate),
Chelate agents (such as phytic acid and ethylenediaminctetraacetate),
Surfactants (such as polyglycerol fatty acid esters, sucrose fatty acid esters, and lecitin).
Vitamins (such as Vitamins A, $B_1$, $B_2$, $B_6$, C, D, E and derivatives thereof),
Amino acids (such as glycine, trimethylglycine, pyrrolidone carboxylate, serine, carnitine, γ-aminobutyric acid, taurine, threonine, asparagine, glutamine, tyrosine, lysine, histidine, arginine, aspartic acid, glutamic acid, ornithine, valine, and leucine),
[0046]   Antiphlogistic agents (such as glycyrrhizinate derivatives, glycyrrhetinate derivatives, salicylic acid derivatives, hinokitiol, zinc oxide, allantoin),
Humectants (urea, casein, soybean peptides, lactobacillus fermentation metabolites, yeast fermentation metabolites, honey, lactoferrin, albumin, hydrolyzed elastin, hydrolyzed keratin, hydrolyzed silk),
Skin lightening agents (such as ascorbic acid glucoside, 3-O-ethylascorbic acid, arbutin, hydroquinone, kojic acid, RUCINOL, tranexamic acid, sodium adenosine-1-phosphate, magnolignan, ellagic acid, retinoid, rutin, resorcinol, cysteine, and glutathione),
[0047]   Various extracts (for example, an *Angelica keiskei* extract, an avocado fruit extract, a *Hydrangea serrata* leaf extract, an *Althaea officinalis* extract, an *Arnica montana* extract, an aloe extract, an apricot extract, an apricot kernel extract, a *Ginkgo biloba* extract, a fennel fruit extract, a turmeric root extract, an oolong tea extract, a rose fruit extract, an *Echinacea augustifolia* leaf extract, a *Scutellaria baicalensis* root extract, a Phellodendron bark extract, a Japanese Coptis extract, a barley extract, a Hypericum extract, a *Labium album* extract, a *Nasturtium officinale* extract, an orange fruit extract, dry seawater products, a seaweed extract, hydrolyzed elastin, hydrolyzed wheat flour, hydrolyzed silk, a chamomilla extract, a *Daucus carota sativa* root extract, an *Artemisia capillaris* flower extract, a Glycyrrhiza extract, a karkade extract, a *Pyracantha fortuneana* fruit extract, a kiwi fruit extract, a cinchona extract, a cucumber fruit extract, guanosine, a *Gardenia florida* extract, a *Sasa veitchii* extract, a *Sophora angustifolia* root extract, a walnut extract, a grapefruit fruit extract, a *Clematis vitalba* leaf extract, a *Chlorella vulgaris* extract, a *Morus alba* extract, a *Gentiana lutea* extract, a black tea extract, an yeast extract, an *Artium lappa* root extract, a fermented rice bran extract, rice germ oil, a *Symphytum officinale* leaf extract, a *Vaccinium vitis-idaea* extract, an Asiasarum root extract, a Saiko (*Bupleurum falcatum*) extract, an umbilical extract, a salvia extract, a *Saponaria officinalis* extract, a bamboo extract, a *Crataegus cuneata* fruit extract, a Zanthoxylum fruit extract, a *Corthellus shiitake* extract, a Rehmannia root extract, a *Lithospermum erythrorhizon* root extract, a Perilla extract, a linden extract, a *Spiraea ulmaria* flower extract, a peony root extract, an *Acous calamus* root extract, a *Betula alba* extract, an *Equisetum arvense* extract, a *Hedera helix* extract, a *Crataegus oxyacantha* extract, a *Sambucus nigra* flower extract, an *Achillea millefolium* extract, a *Mentha piperita* leaf extract, a sage leaf extract, a mallow extract, a *Cnidium officinale* root extract, a *Swertia japonica* extract, a soybean seed extract, a *Zizyphus jujuba* fruit extract, a thyme extract, a green tea extract, a clove flower extract, an *Imperata cylindrica* extract, a *Citrus unship* peel extract, an *Angelica acutiloba* root extract, a *Calendula officinalis* flower extract, a peach kernel extract, a bitter orange peel extract, a *Houttuynia cordata* extract, a tomato extract, a natto (fermented soybean) extract, a carrot extract, a garlic extract, a *Rosa canina* fruit extract, a *Hibiscus sabdariffa* flower extract, an *Ophiopogon japonicus* root extract, a parsley extract, honey, a witch hazel extract, a *Parietaria officinalis* extract, an *Isodonis japonicus* extract, bisabolol, an *Eriobotrya japonica* extract, a coltsfoot extract, a Japanese butterbur extract, a *Poria cocos* extract, a *Ruscus aculeatus* root extract, a grape fruit extract, propolis, a *Luffa cylindrica* extract, a safflower flower extract, a peppermint extract, a *Tilia platyphyllos* flower extract, a *Paeonia suffruticosa* root extract, a *Homulus lupulus* extract, a *Pinus sylvestris* cone extract, a horse chestnut extract, a white arum extract, a *Sapindus mukurossi* peel extract, a *Melissa officinalis* leaf extract, a peach extract, a *Centaurea cyanus* flower extract, a eucalyptus leaf extract, a *Saxifraga sarmentosa* extract, a *Citrus junos* fruit extract, a Coix lacryma-jobi var, ma-yuen seed extract, a Mogwort leaf extract, a lavender extract, an apple fruit extract, a lettuce leaf extract, a lemon fruit extract, an *Astragalus sinicus* extract, a rose extract, a rosemary leaf extract, a *Anthemis nobilis* flower extract, a royal jelly extract, a birch extract, an iris extract, a grape fruit extract, a *Lilium candidum* bulb extract, a *Crocus sativus* flower extract, a *Zingiber officinale* root extract, a *Capsicum annuum* fruit extract, a *Angelica acutiloba* root extract, a seaweed extract, a placental extract, a placenta extract, a *Camomila recutita* extract, a *Coix lacryma-jobi* seed extract, a *Citrus junos* seed extract, a grape seed extract, a watercress extract, an *Epiphyllum oxpetalum* extract, a white lupin extract, a ginger root extract, and a cockscomb extract),

Activator agents (for example, royal jelly, a photosensitizer, and cholesterol derivatives),
Blood circulation promoters (for example, nonylic acid vanillylamide, nicotinic acid benzyl ester, β-butoxyethyl nicotinate ester, capsaicin, zingerone, cantharis tincture, ichthammol, tannic acid, α-borneol, nicotinic acid tocopherol, inositol hexanicotinate, cyclandelate, cinnarizine, tolazoline, acetylcholine, verapamil, cepharanthine, and γ-orizanol),
Antiseborrheic agents (for example, sulfur and thianthol),
Anti-inflammatory agents (for example, tranexamic acid, thiotaurine, and hypotaurine).
One of these active ingredients may be used or two or more of these may be used in combination, depending on the purpose of use of the gel sheet.

[Configuration of gel sheet]

The gel sheet of the invention may consist only of a single hydrogel layer containing the hydrogel described above, or may have a multilayer configuration further including at least one other layer. Alternatively, plural hydrogel layers may be provided. The components described above each may be included in the hydrogel layer(s), or, if possible, may be included in another layer. When there are plural hydrogel layers, the respective components may be included in the same hydrogel layer, or may be grouped and separately included in plural layers. When the gel sheet has a multilayer configuration as described above, the amounts of the respective components in the entire gel sheet may fall within the respective ranges described above.

<Sheet-shaped base material>

For the purpose of reinforcing the hydrogel layer and improving the handleability of the gel sheet, a sheet-shaped base material may be provided in the hydrogel layer or may be provided adjacent to the hydrogel layer.
It is preferable to provide a sheet-shaped base material from the viewpoint of improvement in handleability and dimensional stability of the gel sheet of the invention.

When the sheet-shaped base material is used as a support layer, a known sheet-shaped support material, such as nonwoven cloth, woven cloth, a film, or a gel, may be used.
The material that constitutes the nonwoven cloth or woven cloth is not particularly limited, and generally-used fibers can be used. Example thereof include natural fibers such as cellulose and fibroin, recycled fibers such as rayon, and synthetic fibers such as nylon, polyester, polyethylene, polypropylene, acryl, polylactate, and polyurethane, and cellulose, nylon, and polyester are preferably used.

The woven cloth or nonwoven cloth which is preferably used in the invention has a basis weight of preferably from 3 $g/m^2$ to 100 $g/m^2$, more preferably from 5 $g/m^2$ to 70 $g/m^2$, and particularly preferably from 5 $g/m^2$ to 50 $g/m^2$. When the basis weight is within the above range, the sheet-shaped base material has a strength in a range in which excellent handleability is achieved, while flexibility of the gel sheet is not impaired and adhesion property thereof when adhered does not decrease.

When a film is used, the material thereof may be arbitrarily selected, and examples thereof include liquid-impermeable mono- or multi-layer plastic sheets. Alternatively, sheets having apertures for liquid permeation at a part thereof or the entire surface thereof, porous sheets, and mesh sheets may be used.

The thickness thereof is preferably from about 0.01 mm to 1 mm from the viewpoint of handleability. When the gel sheet is used as a cosmetic preparation or the like, materials having high transparency may sometimes be preferably used as described below.

When the sheet-shaped base material is provided in the hydrogel layer, a liquid-permeable sheet such as woven cloth, nonwoven cloth, a porous sheet, or a mesh is preferable. When the sheet-shaped base material is used as a support at only one surface of the hydrogel layer, a liquid-impermeable sheet or thick woven or nonwoven cloth may be used.

When the sheet-shaped base material is used as a reinforcing layer for the hydrogel layer, the base material to be used may be a polymer gel sheet or a hydrophilic polymer film, each of which has a high rupture strength, such as a crosslinked gel (such as a gelatin/glutaraldehyde crosslinked gel or a polyacrylic acid/polyvalent metal ion crosslinked gel), a physical gel (such as an agarose gel or κ-carrageenan), or a water-insoluble film formed from a hydrophilic polymer (such as a chitosan film, cellophane, or a κ-carrageenan cast film).

Of these, the adhesive gel sheet of the invention particularly preferably has a configuration in which the hydrogel and the woven or nonwoven cloth are integrated. Here, the integrated configuration refers to a configuration in which a base material sheet, such as woven or nonwoven cloth, is superposed on at least one surface of the hydrogel layer such that the base material sheet and the hydrogel layer are tightly adhered to each other at strength at which separation does not occur while the gel sheet is used, or a configuration in which the base material sheet is present within the hydrogel layer in a unseparably integrated manner such that the hydrogel layer is present at both surfaces of the base material sheet.

In the gel sheet of the invention, it is preferable to provide a protection sheet to a surface of the hydrogel layer that is to be applied to a living body, for the purpose of maintaining retention of the active ingredient(s) or water until use. As the protection sheet, a polyethylene film, a polypropylene film, a PET film, or the like is preferably used. In particular, it is preferable to use a polyethylene film or polypropylene film having a thickness of 500 μm or less, and it is more preferable to use a polyethylene film or polypropylene film having a thickness of from 20 μm to 400 μm.

[Method of manufacturing gel sheet]

The gel sheet of the invention may be manufactured by a generally-employed method. For example, when the gel sheet is constituted only from the hydrogel, the gel sheet may be manufactured in accordance with a general method of manufacturing a hydrogel. Specifically, a method of may be employed including:

heating and mixing the respective components to be included in the hydrogel, thereby forming a sol product (hydrogel-forming formulation liquid);

thereafter applying the hydrogel-forming formulation liquid in a sheet shape using a coating apparatus such as a doctor blade; and

leaving the resultant to stand under a normal temperature atmosphere or cooling the resultant, thereby completing gelation and forming a hydrogel layer, which is used as a gel sheet.

In general, the thickness of the hydrogel layer in the gel sheet of the invention is preferably from 0.4 to 2 mm and particularly preferably from 0.5 to 1.5 mm, regardless of the presence or absence of a support, from the viewpoint of shape maintaining property and handleability.

A gel sheet having a support layer may be manufactured by, for example, a method of superposing a hydrogel layer on a surface of a sheet-shaped base material, the method including:

applying the hydrogel layer-forming solution obtained as described above onto a sheet-shaped base material; and performing the same subsequent operations as above, or
a method including:

after the hydrogel layer-forming solution is applied but before the gelation is completed, superposing the sheet-shaped base material on the surface thereof; and

thereafter completing the gelation to integrate the hydrogel layer and the base material.

When a sheet-shaped base material is positioned in the hydrogel layer, a method may be employed which include: applying a hydrogel to have a sheet form, or pouring a hydrogel into a mold and thereafter impregnating the sheet-shaped base material with the hydrogel before the completion of gelation; and completing the gelation.

Active ingredients such as the polyhydric alcohol and/or O/W emulsion may be added separately or at once during the manufacture process of the hydrogel, or may be introduced by impregnating the hydrogel in a solution containing the active ingredients for about 1 to 3 days after the manufacture of the hydrogel.

When a protection sheet is provided, one surface or both surfaces of the gel sheet may be covered with the protection sheet(s) if the gel sheet is formed only of the hydrogel layer, and the protection sheet may be superposed on a surface of a hydrogel layer at which a sheet-shaped base material is not provided if the gel sheet has a multilayer-configuration having the sheet-shaped base material and the hydrogel layer.

[Physical properties of gel sheet]

The adhesive gel sheet for a living body of the invention preferably has a high transparency from the viewpoint of reducing the discomfort feeling on appearance when adhered to the skin.

The transparency of the gel sheet of the invention may be evaluated based on the size of characters that are recognizable when observed through the gel sheet. Regarding the gel sheet of the invention, it is preferable that 12-point characters in Ming typeface can be recognized, and it is more preferable that 10-point characters can be recognized. Within the range, it is easy to confirm the skin condition when the gel sheet is adhered. However, when the gel sheet is applied to a use region where the appearance does not matter, the gel sheet is not necessarily transparent.

When the gel sheet has to be transparent, it is preferable that the sheet-shaped base material, which is used together with the hydrogel layer, is selected from materials having excellent light transparency, such as nylon cloth having a small basis weight or a transparent resin film.

The shape of the gel sheet of the invention when practically used is not particularly limited. The gel sheet may be in the form of a tape wound to form a roll, or may be an independent and isolated sheet. In the case of the isolated sheet, the shape thereof is freely determined, and is appropriately selected in accordance with the purpose of use and the location at which the gel sheet is used. The shape of the gel sheet may be ellipse, circle, cordiform, semicircle, semiellipse, square, rectangle, trapezoid, triangle, or a shape obtained by a combination thereof. Alternatively, a shape conforming to the application location, or a shape allowing a most suitable adhesion, which varies with the location at which the gel sheet is used, may be appropriately designed.

For examples, when the gel sheet is used as an adhesive sheet for a living body, a protruding portion or a recessed portion may be provided at a center or peripheral region of the gel sheet for the purpose of position adjustment or the like, or a slit, a portion having a cut-out, or the like may be arranged to accord the shape of the location at which the gel sheet is used. By adopting such embodiments, the handleability of the adhesive sheet at the application location may be improved, and adhesion to an adjacent adhesion target in an area to which the application of the gel sheet is required may be improved.

The application location and the shape of the gel sheet of the invention are described.

Examples of the application location when the gel sheet of the invention is applied to a living body include face (lips, a cheek area, an eye area, upper and lower areas of eyes, a nose area, a forehead area, or the entire face), an arm area, a leg area, a chest area, a belly area, a back area, and a neck area.

When the gel sheet of the invention is used as an adhesive sheet for a living body, not only the shape, but the area, the thickness, the adhesive properties of the hydrogel outermost surface, and the like may also be appropriately adjusted in accordance with the application location. The kinds and contents of the active ingredients to be contained may appropriately be adjusted.

For example, when forming an adhesive sheet for a living body of which application location is the entire face, it is preferable that the sheet is made to have a shape in which portions corresponding to the positions of eyes and mouth are cut out and a slit is formed at a portion corresponding to nose, and that adjustments, such as increasing the adhesiveness of the adhesive layer or reducing the thickness thereof, are performed in consideration of the largeness of the adhesion area. Alternatively, the shape for the face may be divided into two portions; that is, an upper portion to be applied to the forehead and a region surrounding the eyes and nose, and a lower portion to be applied to an area extending from a region surrounding mouth to chin.

The gel sheets may be sealed in a wrapping material formed from a non-breathable material, in order to prevent decrease in water and active ingredients over time before use.

For example, a continuous gel sheet in a tape shape may be stored in an airtight container that can be opened and closed, for example, a wrapping bag that has a zip and that is formed from a non-breathable sheet, or a container that has a lid and that is formed from a non-breathable resin. In a case of independent and isolated sheets, each of the sheets may be sealed in an openable separate bag formed from a non-breathable sheet. When a gel sheet is stored and distributed in such a state, the water content and active ingredient(s) can be retained in suitable conditions until use.

The gel sheet of the invention having the above configuration has excellent adhesion property to living bodies, excellent moisture retention capability, and excellent syneresis property of active ingredients, and excellent handleability. Therefore, as a result of selections of the kinds and amounts of the components to be contained in the hydrogel layer, the thickness of the hydrogel layer, and the form of the gel sheet, the gel sheet is useful for adhesive skin patches as retainers of transdermally absorbable pharmaceuticals with which drugs are administered to living bodies; cosmetic preparations in sheet form, such as facial masks, that are used for beauty art, facial treatment, skin treatment, or the like; retainers of active ingredients such as skin penetrating components or antiphlogistic analgesic components; and adhesive tapes for living bodies and wound-dressings which are used for protection of wounds, fixation of drugs, and the like.

[Cosmetic preparation in sheet form]

The cosmetic preparation in sheet form of the invention may be formed from the gel sheet of the invention. Specifically, the gel sheet of the invention may be used as the cosmetic preparation if the gel sheet retains, as an active ingredient, at least one of a vitamin, an ammo acid, a moisture retaining component, a skin-lightening component, a fragrance material, an antiseptic, an astringent component, or the like. The cosmetic preparation in sheet form is used in such a manner that the surface of the hydrogel layer is tightly adhered to the skin.

Since the gel sheet of the invention is excellent in handleability and skin penetration property of the active ingredient and the like, the cosmetic preparation in sheet form of the invention is particularly useful as a cosmetic preparation in sheet form such as a facial mask that is adhered to the face to provide moisture and medicinal components to the skin as described above.

The disclosure of Japanese Patent Application Laid-Open (JP-A) No. 2007-284489 is incorporated by reference herein in its entirety.

All publications, patent applications, and technical standards mentioned in this specification are herein incorporated by reference to the same extent as if each individual publication, patent application, or technical standard was specifically and individually indicated to be incorporated by reference.

EXAMPLES

The invention is described in more detail below by referring to Examples. However, the invention is not intended to be limited to these. Hereinafter, when indicating a content or an addition amount, "part(s) by mass" and "% by mass" may be represented by "part(s)" and "%", respectively.

<Preparation of O/W emulsion>

The following components were dissolved for 1 hour while heating at 70°C, whereby a water phase composition was obtained.

(Water phase components)

·Sucrose oleate ester          15 g
·Decaglyceryl monooleate          23 g
·Glycerin          500 g
·Pure water          322 g

Furthermore, the following components were dissolved for 1 hour while heating at 70°C, whereby an oil phase composition was obtained.

(Oil phase components)

·Haematococcus algae extract (content ratio of astaxanthins was 20% by mass)          40 g
·Mix tocopherol          10 g
·Lecithin (derived from soybeans)          90 g

The water phase composition was stirred using a homogenizer (10,000 rpm) while being maintained at 70°C, and the oil phase composition was added thereto, as a result of which an emulsion was obtained. The obtained emulsion was subjected to high pressure emulsification using an ultimizer HJP-25005 (manufactured by Sugino Machine Ltd.) at a pressure of 200 MPa. The volume average particle diameter of the obtained emulsion was measured using a fiber-optics particle analyzer FPAR-1000 (manufactured by Otsuka Electronics Co., Ltd.) at 25°C, and was found to be 90 nm.

<Preparation of hydrogel>

Among the components shown in Table 1, the components except the O/W emulsion obtained as described above were sequentially added to water, and heated and kneaded at 80°C, as a result of which a sol product was obtained. The numerical values indicated under the heading of "hydrogel formulation" in Table 1 represent the contents (unit: % by mass) of the respective components.

After the sol product was cooled to 60°C, the O/W emulsion was added thereto and stirred uniformly. The resultant was spread using a doctor blade to have a thickness of 0.8 mm, then covered with nylon knitted cloth having a basis weight of 20 g/m2, and left to stand at 25°C for 30 minutes, whereby a gel sheet for a living body of Example I was obtained which had the nylon knitted cloth as a sheet-shaped base material and a hydrogel layer provided at both surfaces thereof. The hydrogel layer had a total thickness of 0.8 mm.

[Table 1]

| | | Example 1 | Example 2 | Example 3 | Example 4 | Example 5 | Comparative Example 1 | Comparative Example 2 | Comparative Example 3 | Comparative Example 4 |
|---|---|---|---|---|---|---|---|---|---|---|
| Hydrogel formulation | κ-Carrageenan | 0.5 | 0.5 | 0.5 | 0.5 | 0.5 | 0.5 | 0.5 | | |
| | Sodium polycarylate | | | | | | | | 12 | 12 |
| | Magnesium ascorbyl phosphate | 1 | 1 | 1 | 1 | | | | | |
| | Magnesium chloride | | | | | 0.4 | 0.1 | 0.1 | | |
| | Glucomannan | 0.4 | 0.4 | 0.4 | 0.4 | 0.4 | 0.4 | 0.4 | | |
| | PLURONIC F-127 | 1 | 1 | 1 | 1 | 1 | | | | |
| | 1,3-Butanediol | 10 | 10 | 10 | 10 | 10 | 10 | 10 | 10 | 10 |
| | Synthetic aluminum silicate | | | | 0.05 | | | | 2 | 2 |
| | O/W emulsion | | 0.2 | 0.2 | 0.2 | 0.2 | | 0.2 | | 0.2 |
| | Acid-processed gelatin (swine skin) | 1 | 1 | 1 | 1 | 1 | 1 | 1 | | |
| | Collagen tripeptide | | | 0.1 | | | | | | |
| | Water | 86.1 | 85.9 | 85.8 | 85.85 | 86.5 | 88 | 88 | 76 | 76 |

(continued)

| | | Example 1 | Example 2 | Example 3 | Example 4 | Example 5 | Comparative Example 1 | Comparative Example 2 | Comparative Example 3 | Comparative Example 4 |
|---|---|---|---|---|---|---|---|---|---|---|
| Evaluation results | Syneresis rate (%) | 65 | 62 | 60 | 54 | 49 | 35 | 32 | 16 | 17 |
| | Sense of adhesion | A | A | A | A | A | B | B | A | A |
| | Moisture retention capability | A | A | AA | A | A | B | B | C | C |
| | Skin barrier property | B | A | A | A | A | C | C | C | C |
| | Functional evaluation | A | A | A | A | A | C | C | C | C |
| | Water content ratio (%) | 82 | 82 | 83 | 81 | 84 | 86 | 84 | 73 | 72 |

In Table 1, PLURONIC F-127 (trade name) is a polyether manufactured by BASF.

(Evaluation of gel sheet)

The gel sheets for a living body of Examples I to 5 and Comparative Examples 1 to 4 were used for the following evaluations, and the results of the evaluations were shown in Table 1. The functional evaluations for adhesion degree, moisture retention capability, skin barrier property, and skin beautifying effect were performed in such a manner that the adhesive gel sheet for a living body of Examples 1 to 5 and Comparative Examples 1 to 4 were used by (were adhered to the faces of) 10 monitors, and average values of their evaluations were shown in Table 1.

(1) Syneresis rate

Each of the gel sheets for a living body of Examples 1 to 5 and Comparative Examples 1 to 4 was cut into a size of 3 cm×3 cm, and it was sandwiched between two upper sheets and two lower sheets of filter paper having a diameter of 9 cm, which was filter paper No. 2 available from Advantec Toyo Kaisha, Ltd. [filter paper defined by JIS P3801 (995): 125 g/m$^2$, having a thickness of 0.26 mm, a water filtering time of 80 seconds, and a water absorbency of 80 cm], and left to stand under an atmosphere of 25°C and a relative humidity of 55% for ten minutes. The change in mass of the filter paper was measured to determine the mass of liquid absorbed by the filter paper, and a syneresis rate was calculated in accordance with the following Formula 1. The same test was repeated 5 times, and the average value thereof was shown in Table 1 as the syneresis rate (unit: % by mass).

$$\text{Syneresis rate} = (\text{mass of liquid absorbed by filter paper / initial mass of gel*}) \times 100$$

(Formula 1)

* Here, the initial mass of gel was a value obtained by subtracting the mass of nylon knitted cloth serving as the sheet-shaped base material from the mass of the gel sheet test piece.

(2) Sense of adhesion

A case in which the adhered gel sheet conformed to and was tight contact with the surface of the face was graded A; a case in which the adhered gel sheet partially separated from the face surface was graded B; and a case in which wrinkles occurred at portions and separation from the face surface occurred at many portions was graded C. When the gel sheet was adhered to the skin in the evaluation of the sense of adhesion, rupture or undesired stretching was not observed, which indicates that the gel sheets of Examples 1 to 5 had excellent handleability.

(3) Moisture retention capability

The water content in the horny layer at a tail of the eye of each of the 10 monitors before adhesion of the gel sheet was evaluated in terms of a conductance value obtained using a horny layer water content analyzer (manufactured by Asahi Biomed).
Thereafter, the adhesive gel sheets for a living body of Examples 1 to 5 and Comparative Examples 1 to 3 were adhered to the tails of eyes for 15 minutes, and then removed. Thirty minutes after the removal, the water content in the horny layer at the tail of the eye was measured in the same manner as above. Both values were compared with each other; a case in which an increase in conductance in terms of the average of the 10 monitors was 15% or more was graded AA, a case in which an in crease in the conductance in terms of the average of the 10 monitors was from 10% to less than 15% was graded A, a case in which an increase in conductance in terms of the average of the 10 monitors was from 2% to less than 10% was graded B, and a case in which a change in the conductance in terms of the average of the 10 monitors was less than 2% was graded C.

(4) Skin barrier property

In order to evaluate a skin problem alleviation function, a transepidermal water loss (TEWL) was measured according to the following method.
That is, adhesion of the gel sheet for 15 minutes per day was performed 3 consecutive days. On the third day, a transepidermal water loss (TEWL) immediately after the adhesive gel sheet for a living body was removed was measured using a water evaporation analyzer (manufactured by Asahi Biomed). A case in which the TEWL decreased by 5% by mass or more as compared with the value measured before the test was graded A, a case in which the TEWL decreased by from 1 % by mass to less than 5% by mass as compared with the value measured before the test was graded B, and a case in which a change in TEWL as compared with the value measured before the test was not observed was graded C.

(5) Functional evaluation

Adhesion of the gel sheet for 15 minutes per day was performed for 3 consecutive days. On the third day, impression of the skin appearance immediately after the adhesive gel sheet for a living body was removed was evaluated. A case in which a clear improvement in the fineness of the skin texture was felt was graded A, a case in which a slight improvement in the fineness of the skin texture was felt was graded B, and a case in which a change in the skin appearance was not felt was graded C.

(6) Water content ratio

One gram of the hydrogel layer was taken out of the gel sheet, and dried under reduced pressure at 25°C. The drying was continued until change in mass of the sample became unobservable, and a water content ratio was calculated in accordance with the following Formula 2.

$$\text{Water content ratio (\%)} = [(\text{initial mass} - \text{mass after drying})/\text{initial mass}] \times 100$$

$$\cdots(\text{Formula 2})$$

The measurement test was repeated 5 times, and the average thereof was shown in Table 1 as the water content ratio (unit: % by mass).

As shown in the results in Table 1, the gel sheets of Examples 1 to 5 had excellent syneresis property. Also, it was found that, in practical use, the gel sheets of Examples 1 to 5 had excellent adhesion property to the skin, excellent handleability when adhered to the skin, and excellent improving effects on moisture retention capability and skin barrier property, and had skin beautifying effect whereby the texture of the skin is made fine. In contrast, when the gel sheets of Comparative Examples 1 to 4, which had low syneresis property, were used, the improved effects in, especially, the functional evaluation were not sensed, and moisture retention capability thereof was inferior.

**Claims**

1.  A gel sheet comprising a hydrogel, the hydrogel comprising at least 50 parts by mass of water-soluble divalent metal salt with respect to 100 parts by mass of anionic polymer compound.

2.  The gel sheet according to claim 1, wherein a content of water-soluble divalent metal salt in the hydrogel is from 0.2% by mass to 10% by mass.

3.  The gel sheet according to claim 1, wherein a content of tri- or higher-valent metal salt included in the hydrogel is 0.1% by mass or lower.

4.  The gel sheet according to claim 1, wherein the hydrogel comprises at least one selected from the group consisting of collagen and collagen-degradation products.

5.  The gel sheet according to claim 1, wherein the hydrogel comprises a polyether.

6.  The gel sheet according to claim 1, wherein the hydrogel comprises an oil/water emulsion.

7.  The gel sheet according to claim 1, wherein the hydrogel comprises a polysaccharide.

8.  The gel sheet according to claim 1, wherein the hydrogel comprises a polyhydric alcohol compound.

9.  The gel sheet according to claim 1, wherein the hydrogel has a water content ratio of from 70 to 95% by mass.

10. The gel sheet according to claim 1, wherein the gel sheet comprises: a hydrogel layer comprising the hydrogel; and a sheet-shaped base material that is arranged within the hydrogel layer or adjacent to the hydrogel layer.

11. The gel sheet according to claim 10, wherein the hydrogel layer has a thickness of from 0.4 to 2 mm.

12. A cosmetic preparation in sheet form, comprising the gel sheet according to any one of claims 1 to 11.

## INTERNATIONAL SEARCH REPORT

| International application No. |
|---|
| PCT/JP2008/068752 |

**A. CLASSIFICATION OF SUBJECT MATTER**
See extra sheet.

According to International Patent Classification (IPC) or to both national classification and IPC

**B. FIELDS SEARCHED**

Minimum documentation searched (classification system followed by classification symbols)
A61K8/00-99, A61K9/70, A61K31/33-33/44, A61K47/00-48, A61L15/16,
A61L15/58, A61P17/16, A61Q1/00-99/00

Documentation searched other than minimum documentation to the extent that such documents are included in the fields searched
Jitsuyo Shinan Koho          1922-1996   Jitsuyo Shinan Toroku Koho   1996-2008
Kokai Jitsuyo Shinan Koho    1971-2008   Toroku Jitsuyo Shinan Koho   1994-2008

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 3-223213 A (Sekisui Chemical Co., Ltd.), 02 October, 1991 (02.10.91), | 1-5,7,8,10, 12 |
| Y | Page 1, lower right column, lines 11 to 13; page 3, upper left column, line 5 to page 5, upper left column, line 8; example 1 (Family: none) | 6,8-11 |
| X | JP 2005-23040 A (Okayama Taiho Yakuhin Kabushiki Kaisha), 27 January, 2005 (27.01.05), | 1,2,4,5,7,8, 10,12 |
| Y | Par. Nos. [0020], [0021], [0025], [0028]; examples 1 to 5 (Family: none) | 6,8-11 |

☒ Further documents are listed in the continuation of Box C.       ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered   to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 25 December, 2008 (25.12.08) | 13 January, 2009 (13.01.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

| International application No. |
| --- |
| PCT/JP2008/068752 |

C (Continuation).  DOCUMENTS CONSIDERED TO BE RELEVANT

| Category* | Citation of document, with indication, where appropriate, of the relevant passages | Relevant to claim No. |
| --- | --- | --- |
| X<br>Y | JP 2003-277253 A  (Kobayashi Pharmaceutical Co., Ltd.),<br>02 October, 2003 (02.10.03),<br>Claim 1; Par. Nos. [0001], [0015]; examples<br>(Family: none) | 1-3,7,9,12<br>4-6,8-11 |
| Y | JP 2004-292345 A  (Sekisui Plastics Co., Ltd.),<br>21 October, 2004 (21.10.04),<br>Par. No. [0030]<br>(Family: none) | 6 |
| Y | JP 2005-320264 A  (Dia Pharmaceutical Co., Ltd., Kanebo, Ltd.),<br>17 November, 2005 (17.11.05),<br>Claims 1, 7; Par. No. [0034]<br>(Family: none) | 9,11 |

Form PCT/ISA/210 (continuation of second sheet) (April 2007)

**INTERNATIONAL SEARCH REPORT**

International application No.

PCT/JP2008/068752

Continuation of A. CLASSIFICATION OF SUBJECT MATTER
  (International Patent Classification (IPC))

A61K8/73(2006.01)i, A61K8/02(2006.01)i, A61K8/19(2006.01)i,
A61K8/20(2006.01)i, A61K8/34(2006.01)i, A61K8/65(2006.01)i,
A61K8/67(2006.01)i, A61K8/86(2006.01)i, A61K9/70(2006.01)i,
A61K33/06(2006.01)i, A61K47/10(2006.01)i, A61K47/34(2006.01)i,
A61K47/36(2006.01)i, A61K47/42(2006.01)i, A61L15/16(2006.01)i,
A61L15/58(2006.01)i, A61P17/16(2006.01)i, A61Q19/00(2006.01)i

(According to International Patent Classification (IPC) or to both national
classification and IPC)

Form PCT/ISA/210 (extra sheet) (April 2007)

## INTERNATIONAL SEARCH REPORT

International application No.

PCT/JP2008/068752

**Box No. II    Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:
   See extra sheet

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**
the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

| INTERNATIONAL SEARCH REPORT | International application No. |
|---|---|
| | PCT/JP2008/068752 |

Continuation of Box No.III of continuation of first sheet(2)

For the reasons stated below, this international application is considered to contain seven inventions which do not satisfy the requirement of unity of invention.

Main invention: claims 1, 2, 8, 10-12
Second invention: claim 3
Third invention: claim 4
Fourth invention: claim 5
Fifth invention: claim 6
Sixth invention: claim 7
Seventh invention: claim 9

The technical feature common to the main invention and the second to seventh inventions is "a gel sheet containing a hydrogel which is produced by using not less than 50 parts by mass of a water-soluble divalent metal salt per 100 parts by mass of an anionic polymer compound".

It has been, however, revealed that this technical feature is not novel since it is disclosed in document 1: JP 3-223213 A (Sekisui Chemical Co., Ltd.), 2 October, 1991 (02.10.91) or document 2: JP 2005-23040 A (Okayama Taiho Yakuhin Kabushiki Kaisha), 27 January, 2005 (27.01.05).

Consequently, the technical feature cannot be considered as a "special technical feature" within the meaning of PCT Rule 13.2, second sentence, since it makes no contribution over the prior art. It is therefore considered that there is no technical relationship between the main invention and the second to seventh inventions involving one or more of the same or corresponding special technical features.

Consequently, the main invention and the second to seventh inventions do not satisfy the requirement of unity of invention.

Form PCT/ISA/210 (extra sheet) (April 2007)

**EP 2 210 583 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3081213 A
- JP 2005225837 A
- JP 2003183147 A
- JP 2005075817 A
- JP 2007284489 A